# EUROPEAN PATENT APPLICATION

(11) **EP 4 768 567 A1**
(43) Date of publication of application: **01.07.2026**
(21) Application number: 25216653.3
(22) Date of filing: 18.11.2025
(51) Int. Cl.: C12M 1/24, C12M 1/00, C12M 1/12, B01L 3/00, C12M 1/26

(54) **CONTAINER FOR BIOLOGICAL MATERIAL**

(30) Priority: 24.12.2024 EP 24223152
(71) Applicant: TPP Techno Plastic Products AG, 8219 Trasadingen (CH)
(72) Inventor: Tanner, Rolf, 8224 Löhningen (CH)
(74) Representative: Keller Schneider Patent- und Markenanwälte AG

(57) **Abstract**

The cap (2) for closing an opening (1.3) of a container (1) for biological material, has a closure face (2.2) suitable for covering an opening (1.3) of a mating container (1). The closure face (2.2) is designed to separate an outside area (11) from an inside area (10) of the container (1). The closure face (2.2) has a septum (3), the septum (3) providing a flexible port area (3.2), wherein the port area (3.2) is designed to be opened by a conduit (4). The flexible port area (3.2) is shaped as a dome protruding towards the outside area (11).

## Description

### Technical field

The invention relates to disposable containers for processing biological materials in a highly automated and rapid manner while maintaining sterility. In particular, the invention refers to so called bioreactors with septa for insertion and removal of samples. The bioreactor may also have a gas permeable membrane for gas exchange with the extemal environment.

### Background art

US 2009/0148941 A1 (Pete Florez et al.) discloses a cell culture system comprising a container having cylindrical side wall, a closed bottom end and an open top end. The cap of the container has a septum and a gas permeable membrane. The septum provides a self-sealing access to the inside of the container after penetration with a syringe. The septum is a disk, i.e. a flat circular sheet of a flexible material. The septum has a port for a syringe and it is fixed in the rigid structure of the cap by means of a retainer ring.

WO 2020/252243 (Becton Dickinson) discloses a pierceable cap for tube shaped containers for biological specimens. The cap has a septum which may have different shapes. In one embodiment the septum is a flat disk on top of which there are extension bodies that help to break the septum when the syringe is inserted to extract content from the container. In another embodiment, the disk-shaped septum is provided with a bulging rim for fixing the septum between the cap and the bottleneck of the container when the cap is screwed on the container tube. The disadvantage is that the septum is only securely fixed when the cap is on the container because the septum is squeezed between the vessel rim and the cap. In a further embodiment the septum has the shape of a cone directed and protruding towards the inside of the vessel.

US 2012/279968 (ANDWIN) discloses a cap for a vessel that is turned upside down during entering of the syringe. The port has a concave area at the inside while the outside is flat.

US 10,266,316 (Levy Abner) and US 2022/119173 (Levy Abner) disclose a cap with a septum that is substantially flat. In the center of the septum the thickness is reduced by a concave area at the outside of the septum surface.

US 2009/257922 A1 discloses a cap with a septum that may be pressure-fitted unto the cap in a way that pushes up a polytetrafluoroethylene layer, creating a dome. In some embodiments. The septum may be dome-shaped in the absence of an attached tube e.g., at atmospheric pressure. The septum dome may be configured and arranged to protrude from a tube. The dome may be slight (e.g., nearly flat) or more pronounced (e.g., hemispherical).

The caps proposed in the prior art are not satisfactory. In particular, the ports do not deal with the risk that liquid substance remaining at the outside of the port may be sucked into the vessel over time.

### Disclosure of invention

The problem to be solved is therefore to achieve a better protection of the container content against contamination during repeated removal of probes from the inside. In particular, the port is to be designed in such a way that contaminated liquid on the outside is repelled.

The solution of the problem is defined by the elements of claim 1. According to the invention a cap suitable for closing an opening of a container for cell cultures is characterized by the following elements:
a) the cap has a closure face suitable for covering an opening of a container, wherein the closure face is designed to separate an outside area from an inside area of the container;
b) the closure face has a septum and the septum provides a flexible port area, wherein the port area is designed to be opened by a conduit (e.h. the tip of a syringe); entering from the outside area to the inside area and wherein the port is designed to remain closed in absence of a conduit;
c) the flexible port area is shaped as a dome protruding towards the outside area.

The advantage is that any liquid on the outside surface of the septum flows away from the dome-shaped port towards the edge of the dome. And this takes place in an upright position of the container.

The closure face of the cap is defined by the particular wall (of the cap), which is designed to cover the opening, when the cap is fixed to the container. The closure face may be surrounded by a side wall of the cap. The side wall is not part of the closure face, but it is adjacent to the closure face.

The closure face consists of at least a rigid (generally planar) structure and the septum. The closure face may also have a gas membrane.

The cap inherently defines an outside area and an inside area. The inside area is the area that will be inside the container when the cap is fixed to the container. The outside area is the area that will be outside of the container when the cap is fixed to the container. The closure face is designed to separate the outside area from the inside area.

The septum is integrated in the closure face. As a consequence it also separates the outside area from the inside area.

The septum has a flexible port area. The port area is designed to be opened by a conduit, That is, the conduit penetrates the flexible port area when it is pushed towards the inside area of the container. When no conduit penetrates the port, the port remains closed. That means, the septum has a resting state, in which the port area is closed.

The port area is defined by a slit in the septum. According to the invention, the port area is the area within a the smallest polygon, that can be drawn around the slit, so that the slit is completely within the polygon. Example: If the slit has the shape of a cross with four arms of equal length, the port area is a square connecting the outer ends of the four arms. Another example: if the slit is a star with 5 or 6 arms, the port area is a pentagon or hexagon having its corners at the outer ends of the 5 or 6 arms respectively.

The dome defines a convex surface on the outside. The convex surface of the dome may be a spherical surface. However, it may also be elliptic, oval or otherwise shaped. The key feature is that the inclination of the convex surface points towards the periphery of the dome, so that liquid will flow away to the periphery of the septum.

Generally speaking, the invention also refers to a container with a cap. The cap has the characteristics described above.

### Particular embodiment no. 2

According to a particular embodiment, the flexible port area has a concave shape at the inside area. So, the septum forms at the inside a hollow volume of the closure face. So the outwardly bulging outside surface of the dome is at least partially matched by a concave inside shape. This has the advantage, that flaps provided in the port area of the septum have a better flexibility. Also, it is possible to provide different septa with different port flexibility without having to change the outside surface of the dome. So the optimal drainage of the liquid at the outside of the septum is not impaired when adapting the flexibility of the port area.

In an alternative embodiment, the flexible port area may be flat at the inside area. This might be an advantage if the height of the dome is relatively small.

### Particular embodiment no. 3

According to a particular embodiment the closure face has a central opening in a center of the closure face. Said central opening has a receiving channel that is open towards a center of the central opening. The open side of the receiving channel being oriented along (i.e. arranged in) a plane parallel to the closure face. The receiving channel is accessible from a radial direction emanating from said center of the central opening. The septum has a peripheral rim fixed in the receiving channel of the central opening.

### Particular embodiment no. 4

According to a particular embodiment the peripheral rim has a thickness at least twice of a thickness of the septum in the port area.

### Particular embodiment no. 5

According to a particular embodiment, the flexible port area is designed in the style of a sheet. That is, the flexible port area has a substantially constant thickness t_s throughout its extension. The advantage is, that the port area may easily be manufactured from a sheet of flexible material, such as from a sheet of polysiloxane (silicone).

Preferably the whole septum has a substantially constant thickness.

### Particular embodiment no. 6

According to a particular embodiment, the port area has a radius of curvature r_d relative to a diameter d_p of the flexible port area according to the formula: r_d > d_p/2. The radius of curvature may be different at different locations in the port area. According to the present preferred embodiment the radius of curvature in the port area is always larger than half the diameter of the flexible port. So, there are no sharp edges.

Outside the port area the septum may have a local radius of curvature that is smaller than the half of the diameter of the port area.

The diameter of the port area indicates the diameter of the smallest circle that completely encloses the port area. For instance, the diameter of a square port area is the diagonal of the square.

Preferably, the radius of curvature in the port area is at least 5 times as big as half the diameter d_p of the port area. A large radius of curvature has the advantage that the resistance of the port to conduit in the penetrating forward movement is reduced.

Alternatively the radius of curvature may be smaller at some places of the port area. This means there may be sharp edges along some lines in the port area.

### Particular embodiment no. 7

According to a particular embodiment, the septum is a body of silicone (polysiloxane). Silicone material has a high flexibility.

Preferably the body has a circular shape. The circular shape has the advantage that the septum is easy to mount in the closure face of the cap.

Alternatively, the septum may have an oval shape, a rectangular shape or a different shape.

### Particular embodiment no. 8

According to a particular embodiment, the sheet body has a peripheral rim with a bulge. The bulge protruding substantially orthogonal to a plane defined by the periphery of the sheet body. The bulge has the advantage that the septum can be mechanically fixed in the closure face of the cap. It improves the fixation in the septum in the receiving channel.

An alternative is to avoid any bulging area at the rim and to anchor the septum in the closure face by gluing.

### Particular embodiment no. 9

According to a particular embodiment, the septum has a transition section between the rim and the port area. The transition section bridges the distance between the inner end of the circumferential rim and the outer edge of the port area.

Preferably, the transition section also bridges a distance in z-direction (wherein the z-direction is orthogonal to the geometric plane defined by the rim).

This has the advantage that port area is at an axial distance from the place where the rim of the septum is fixed in the closure face. A further advantage is, that the contaminated liquid that may remain on the septum after retraction of the conduit, can flow further away from the port area.

The transition section may be in part cylindrical and in part conical.

Most preferably, the cross-section follows a smoothly curved line connecting the radial inner end of the rim with the radial outer end of the port area.

Preferably, the transition section extends in a z-direction from the rim towards the outside area of the container.

More preferably, the transition section and the dome are in combination convex towards the outside area of the cap.

Alternatively, the septum may be without a cylindrical or conical section

### Particular embodiment no. 10

According to a particular embodiment, the dome has a height that is not more than 1/5 of a diameter of the dome. This means that the dome is relatively low. The advantage is, that the dome does not take a lot of space in z direction (a direction orthogonal to the closure face).

### Particular embodiment no. 11

According to a particular embodiment, the port area is defined by slits in the septum, the slits dividing the port area into flaps that give way to a conduit that is inserted . Preferably, the slit has the shape of a star with a number n of arms. There are at least three arms starting from a center of the port area and extending in a radial direction. The slit may have four, five, six or even more arms. The advantage is, that the port is easy to manufacture.

Preferable, there are four slits that separate the port area into four flaps.

### Particular embodiment no. 12

According to a particular embodiment, the cap has a peripheral wall oriented orthogonal to the closure face. The peripheral wall is fixed to the closure face and is running along a peripheral edge of the closure face. The peripheral wall has structural elements to mechanically fix the cap at the container opening. The advantage is, that the cap is easy zu manufacture and easy to handle.

Preferably, the axial cross-section of the cap is a u-shape, the two legs of the u-shape representing the peripheral wall running along the peripheral edge of the closure face..

### Particular embodiment no. 13

According to a particular embodiment, the closure face has a rigid structure. The rigid structure has an opening limited by a surrounding edge. The septum is fixed in the opening.

According to a most preferred embodiment, the peripheral rim of the septum is fixed in a u-shaped receiving channel provided in the surrounding edge of the opening.

Alternatively, the septum may be glued to the edge of the opening.

### Particular embodiment no. 14

According to a particular embodiment, the u-shaped receiving channel is open towards a center of the opening. This has the advantage that the septum is safely fixed in z direction (i.e. a direction orthogonal to the closure face). When combining the present embodiment with the embodiment no. 6, the septum is clamped between the two side walls of the receiving channel.

### Particular embodiment no. 15

According to a particular embodiment, the rigid structure of the cap is made of a stiff plastic material, preferably of a polyethylene. The rigid structure has the advantage that the cap may be easily handled by robots.

The rigid structure may also be used to support a gas membrane to vent the inside of the container.

### Particular embodiment no. 16

According to a particular embodiment, the container has cylindrical walls having structural elements for mechanically fixing the cap at the container opening. Preferably, the container is a cylindrical tube. The bottom of the tube can be flat or conical.

### Particular embodiment no. 17

According to a particular embodiment,, the structural elements of the container are arranged on the outside of the container to provide a screw cap fixation of the cap. The structural elements may be ribs or recesses. This has the advantage that the mechanical fixation is easy and yet safe.

Alternatively the structural elements of the container my be ribs or recesses providing a snap closure.

Further preferred embodiments become evident from the following description.

### Brief description of the drawings

The drawings for illustrating preferred examples of the invention show the following:
- Fig. 1a, 1b: a side view of a container with cap;
- Fig. 1c: a perspective view with a cross-section of the container;
- Fig. 2a - 2c: a top plan view, a perspective view and a cross-section of a the rigid structure of the cap;
- Fig. 3a - 3c: a top plan view and a perspective view with a cross-section of the cap;
- Fig. 4a - 4c: a perspective view and a top plan view and a cross-section of the septum;
- Fig. 5a - 5c: a cross-section of the conduit entering through and retracting from the cap;
- Fig. 6: a cross-section of the peripheral rim anchored in the receiving channel of the cap.

In principle, identical parts in the figures are provided with identical reference symbols.

### Ways to carry out the invention

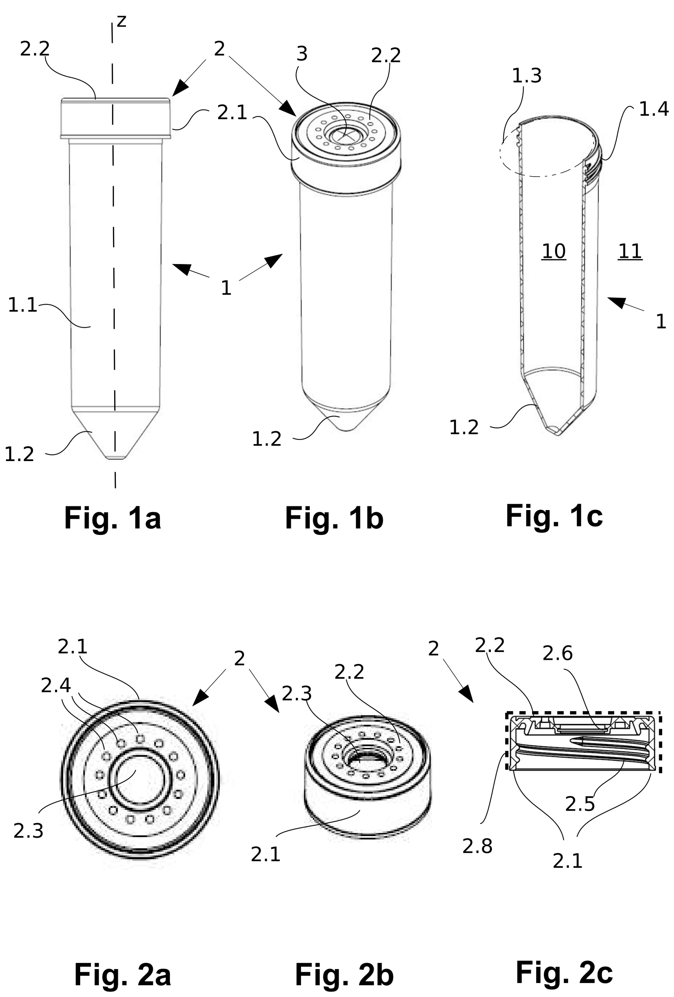

**Figs. 1a and 1b** show a preferred embodiment of the invention. The container 1 has a substantially cylindrical shape. That is, the side wall 1.1 of the container is cylindrical. The cross-section (orthogonal to the longitudinal axis of the container) of the container 1 is in the present embodiment circular. The bottom 1.2 of the container 1 has a conical shape. The top end of the container 1 provides a circular opening 1.3 (see Fig. 1c).

The container 1 is preferable made of a transparent plastic.

The cap 2 has a cylindrical side wall 2.1 and a closure face 2.2. The closure face 2.2 is substantially a circular disk. In the center of the closure face 2.2 there is the septum 3.

The side wall 2.1 of the cap 2 has structural elements at the inner side that cooperate with structural elements 1.4 on the outer side of the side wall 1.1 of the container 1. In the present embodiment the structural elements are screw ribs 1.4 running along a screw line. That means that the cap 2 can be safely screw on the top end of the container 1 to close the opening 1.3.

According to a preferred embodiment of the invention, the side wall 1.1 of the container 1 is inside the side the cylindrical side wall 2.1 of the cap 2 when the cap 2 is fixed to the container 1.

The container of Fig. 1a - 1c is in line with the particular embodiment no. 14 and no. 15.

**Fig. 1c** illustrates the circular opening 1.3 at the top end of the container 1. The screw ribs 1.4 are at the top end of the cylindrical side wall 1.1. The container 1 and the cap 2 separate an inside area 10 of the container and the cap from an outside area 11 of the container and the cap. In the inside area 10 there is e.g. the biological material. In the outside area 11 there may be the rack that holds the container in a desired position.

**Fig. 2a - 2c** show the rigid structure of the cap 1 before mounting the septum and the gas membrane. In the center of the disk shaped closure face 2.2 is the central opening 2.3 for mounting the septum. There are also several peripheral openings 2.4 behind which a gas membrane will be mounted. This illustrates the particular embodiment no. 11.

In line with the preferred embodiment no. 10 the cross-section of the cap 2 is substantially u-shaped: The cylindrical side wall 2.1 defines the two legs of the u-shape 2.8 and the closure face 2.2 defines the bridge of the u-shape 2.8. The u-shape 2.8 is illustrated by a dotted line.

The rigid structure of the cap is made by injection molding. The material is preferably polyethylene. This is in line with particular embodiment no. 13.

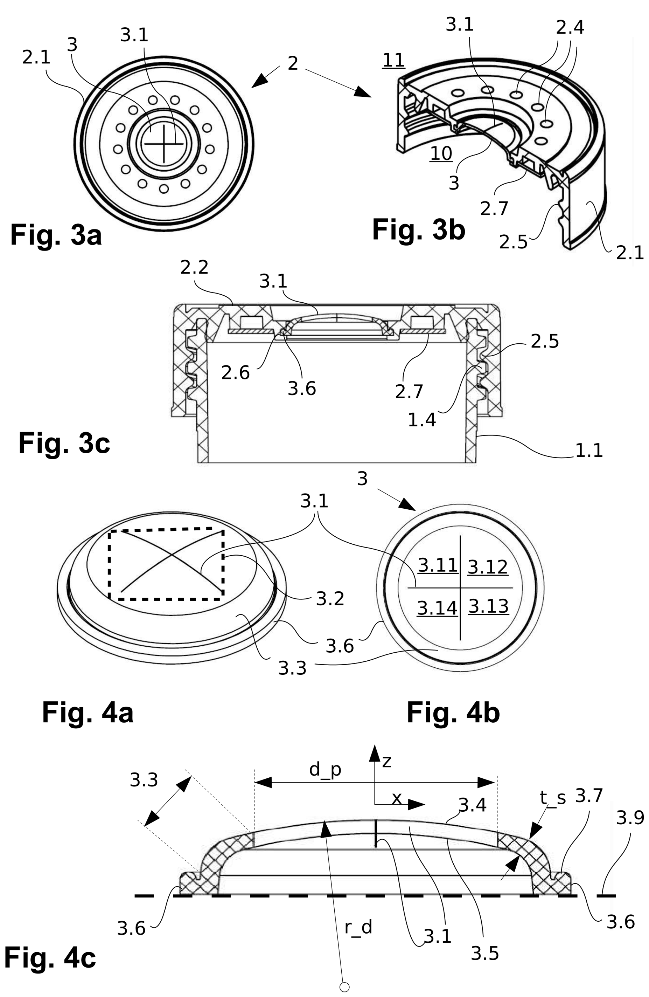

**Fig. 3a - 3c** illustrate the cap 2 with the septum 3. The septum 3 has a cross-shaped slit 3.1. The cross-shaped slit corresponds to a star with four arms of equal length. This is an example for the particular embodiment no. 9.

**Fig. 3c** shows that the rim 3.6 of the septum is fixed in a receiving channel 2.6 of the central opening 2.3. The receiving channel 2.6 is open towards a center of the central opening 2.3. Therefore, the open side of the receiving channel 2.6 is oriented along the plane of the closure face and is accessible from a radial direction emanating from the center of the central opening. Preferably the receiving channel 2.6 has a substantially u-shaped cross-section. The present receiving channel 2.6 is in line with the particular embodiment no. 12.

At the inside of the cap 2 the peripheral openings 2.4 are covered by a gas membrane 2.7. The gas membrane 2.7 may have the plan view of a ring running at a radial distance around the central opening 2.3. It has also a radial distance from the cylindrical side wall 2.1.

The gas membrane 2.7 is separate and different from the septum 3.

**Fig. 4a - 4c** illustrate the septum 3. According to the preferred embodiment no. 5 of the invention the septum 3 is a piece made of polysiloxane. It has a circular shape and therefore a ring-shaped rim 3.6. The central section of the septum 3 forms a port area 3.2 (illustrated by a dotted line). The port area 3.2 is defined by the extension of the slit 3.1. According to the invention the port area is defined as the area within a closed polygon that is generated by connecting the exterior ends of the slit 3.1.

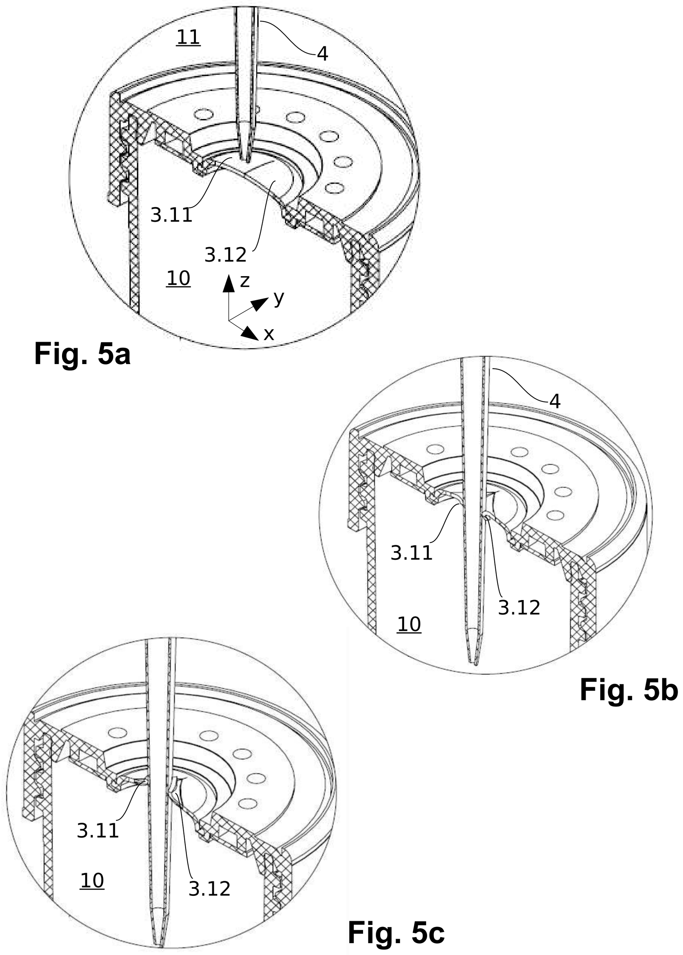

The slit 3.1 has four arms. The consequence is that the port area 3.2 is divided into four flaps 3.11, ..., 3.14. Because the septum 3 is made of a flexible material, the flaps 3.11, ..., 3.14 (which are triangular in the present case) may swing like a door when the conduit is inserted (see Fig. 5a - 5c).

**Fig. 4c** shows the septum in its upright orientation: The dome is protruding upwardly (in z-direction). This is the orientation, when the cap is ready to be screwed on the top end of the container (the container being in an upright position). The ground plane 3.9 of the peripheral rim 3.6 is the geometric plane on which the rim 3.6 rests.

According to a preferred embodiment the rim 3.6 has the shape of a ring defining the outermost edge of the septum 3.

A transition section 3.3 connects the rim 3.6 with the port area 3.2. This transition section 3.3 extends upwardly away from the ground plane 3.9 and inwardly to the port area 3.2. The transition section 3.3 may be in part cylindrical and in part conical. The cross-section may follow a smoothly curved line connecting the radial inner end of the rim 3.6 with the radial outer end of the port area 3.2. The transition section 3.3 is within the framework of the particular embodiment no. 7.

The port area 3.2 is dome-shaped. According to a preferred embodiment the outer surface of the dome is substantially spherical. The radius r_d of curvature of the port area 3.2 is approximately ten times the diameter d_p of the port area 3.2. This is within the framework of the preferred embodiment no. 4.

The port area, therefore, has a convex side 3.4 pointing towards the outside area11 and a concave side 3.4 at the inside area 10. The concave shape defines a concave volume of the septum. This corresponds to the particular embodiment no. 2.

The diameter of the septum 3 is e.g. 10 mm. The dome in the port area has a height of e.g. 1 - 2 mm. This means that the present embodiment is within the limits of the particular embodiment no. 8.

As illustrated by **Fig. 4c****.** the radius of curvature of the septum 7 outside the port area may be smaller than half the diameter of the port area. For instance, in the transition section 3.3 there is clearly a local radius of curvature that is small compared to the diameter of the port area.

The septum is a sheet body and the rim has a bulge 3.7 at a peripheral rim 3.6 of the sheet body, the bulge 3.7 protrudes substantially in z-direction, i.e. orthogonal to a plane 3.9 defined by the periphery of the sheet body. The bulge 3.7 improves the fixation in the receiving channel 2.6. This is in line with the particular embodiment no. 6 and no. 11.

The septum is designed in the style of a sheet. It has a constant thickness t_s. This corresponds to the preferred embodiment no. 3.

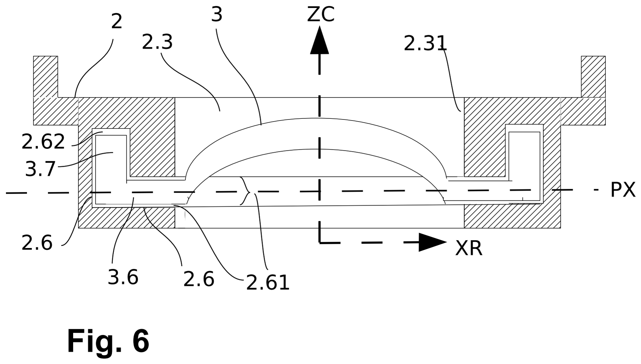

**Fig. 6** illustrates the preferred embodiment with the rim to achieve optimal anchoring of the septum 3 in the cap 2. Fig. 6 only shows the central part of the cap 2, the cylindrical side wall 2.1 and the peripheral openings 2.3 (see Fig. 2a - 2c) are not shown for simplicity of the illustration. The central axis ZC is orthogonal to the closure face (2.2 in Fig. 1b and 1c) and it indicates the center of the central opening 2.3 of the cap 2.

According to a preferred embodiment the peripheral side wall 2.31 limiting the central opening 2.3 has the shape of a circular cylinder.

The side wall 2.31 has a receiving channel 2.6. In the present embodiment, the receiving channel 2.6 continuously runs around the central axis ZC and defines a groove. The opening of the receiving channel 2.6 is directed towards the central axis ZC. Therefore, the receiving channel 2.6 is accessible in a radial direction XR from the central axis ZC.

The open side (opening 2.61) of the receiving channel 2.6 is arranged in a plane PX parallel to the closure face (see closure face 2.2 in Fig. 2c).

The receiving channel 2.6 has an anchoring cavity 2.62 behind the opening 2.61. The anchoring cavity 2.62 has an enlarged cross-section compared to the opening 2.61 of the receiving channel. In particular,
- the cross-section of the anchoring cavity 2.62 measured parallel to the central axis ZC
   is larger than
- the cross-section of the opening 2.61 of the receiving channel measured parallel to the central axis ZC.

Preferably, the cross-section of the anchoring cavity 2.62 measured parallel to the central axis ZC is at least twice as large as the opening 2.61 of the receiving channel measured parallel to the central axis ZC.

The septum 3 has a rim 3.6 which is arranged in the receiving channel 2.6. The rim 3.6 has a bulge 3.7 extending parallel to the central axis ZC. The bulge 3.7 has a cross-section measured parallel to the central axis ZC that is larger than the cross-section of the rim 3.6 measured parallel to the central axis ZC.

According to a preferred embodiment, the septum has substantially a constant thickness throughout the septum area inside the circular area defined by the bulge 3.7.

In any case the bulge 3.7 has a cross-section measured parallel to the central axis ZC that is substantially larger than the cross-section of the opening 2.61 of the receiving channel measured parallel to the central axis ZC. Preferably the cross-section of the bulge 3.7 is at least twice as large as the cross-section of the rim 3.6 measured parallel to the central axis ZC.

It now is evident that the rim 3.6 is safely anchored in the central opening of the cap. Making the bulge 3.7 twice as large as the opening of the receiving channel ensures that even if using a flexible material such as silicone for the septum, the anchoring is safe during the operation illustrated in Figs. 5a-c.

In the present embodiment the enlarged cross-section is rectangular. However, the cross-section may be oval. Or it may have some other convex shape.

In the present embodiment the anchoring cavity only extends to one side of the opening of the receiving channel (namely upwards in Fig. 6, i.e. towards the closure face of the cap). However, the anchoring cavity may alternatively extend to both sides of the opening (upward and downward in Fig. 6) or only downward (in Fig. 6, i.e. away from the closure face).

**Figs. 5a - 5c** illustrate the process of taking samples from the biological material in the container. The conduit 4 (which is e.g. the tip of a syringe) is approaching from above (z direction with respect to the cylindrical cap and the container). The septum 3 is in its resting position which means that dome of the port area is bulging outwardly (with respect to the z direction) towards to the conduit 4.

When the conduit 4 is moved downwardly against z-direction it penetrates the septum 3. In fact the flexible flaps 3.11, 3.12 are bending towards the inside area 10 so that the conduit has a free way to enter through the port area. In this phase the flaps 3.11, 3.12 are oriented against the z-direction.

After the probe (at the bottom of the container) has been aspirated by the conduit 4, the conduit 4 is withdrawn from the inside area as shown in **Fig. 5c****.** As there is some friction between the flaps 3.11, 3.12 and the conduit 4, the flexible flaps switch their orientation and they point in upward direction (i.e. they point in z-direction). When the conduit 4 is completely withdrawn from the septum, the flaps go back to the resting position and close the port area. Any fluid that is remaining on the outside surface of the dome is flowing away from the dome to the transition section 3.3. As there is no slit in the transition section 3.3 there is no risk that the fluid inadvertently penetrates through the slit 3.1 to the inside area.

The embodiments shown in the figures may be modified without leaving the scope of the invention. For example the following modifications are possible:
- Instead of having a bulge on the rim of the septum, providing an indentation in the rim of the septum for co-operating with a rib in the receiving channel 2.6;
- Providing a higher thickness of the septum outside of the port area compared to the thickness inside the port area;
- Providing a non-constant thickness of the septum in the port area (e.g. a thickness that is reduced towards the center of the port area);
- Providing the rim directly adjacent to the port area; i.e. providing no transition section between the rim and the port area;
- Providing a square opening for the septum instead of a circular central opening;
- Providing no peripheral openings 2.3;
- Providing a cap that is inserted into the opening, so that the side wall of the cap is inside the cylindrical side wall of the container, when the cap is fixed to the container;

The invention is not limited to caps and containers for biological material. It may also be used for non-biological chemicals.

### List of reference numerals

- 1: container
- 1.1: side wall
- 1.2: bottom
- 1.3: opening
- 1.4: screw ribs
- 2: cap
- 2.1: cylindrical side wall
- 2.2: closure face
- 2.3: central opening
- 2.31: side wall
- 2.4: peripheral openings
- 2.5: screw ribs
- 2.6: receiving channel
- 2.61: opening of receiving channel
- 2.62: anchoring cavity of receiving channel
- 2.7: gas membrane
- 2.8: u-shape
- 3: septum
- 3.1: slit
- 3.2: port area
- 3.3: transition section
- 3.4: convex side
- 3.5: concave side
- 3.6: rim
- 3.7: bulge
- 3.9: ground plane of the peripheral rim
- 3.11 - 3.14: (triangular) flap
- 10: inside area
- 11: outside area
- PX: plane
- XR: radial direction
- ZC: central axis

## Claims

1. A cap (2) suitable for closing an opening (1.3) of a container (1) for biological material, wherein:
a) the cap (2) has a closure face (2.2) suitable for covering an opening (1.3) of a mating container (1), wherein the closure face (2.2) is designed to separate an outside area (11) from an inside area (10) of the container (1);
b) the closure face (2.2) having a septum (3), the septum (3) providing a flexible port area (3.2), wherein the port area (3.2) is designed to be opened by a conduit (4), in particular a syringe, entering from the outside area (11) to the inside area (10) and wherein the port area (3.2) is designed to remain closed in absence of a penetrating conduit (4);
**characterized in that**
c) the flexible port area (3.2) is shaped as a dome protruding towards the outside area (11).

2. A cap according to claim 1, wherein the flexible port area (3.2) has a concave shape at the inside area (10).

3. A cap according to any of the preceding claims, wherein the closure face (2.2) has a central opening (2.3) in a center of the closure face (2.2), said central opening (2.3) having a receiving channel (2.6) that is open towards a center of the central opening (2.3), an open side of the receiving channel (2.6) being oriented along a plane of the closure face (2.2) and being accessible from a radial direction emanating from said center of the central opening (2.3) and wherein the septum has a peripheral rim (3.6) fixed in the receiving channel (2.6) of the central opening (2.3).

4. A cap according to claim 3, wherein the peripheral rim (3.6) has a thickness at least twice of a thickness of the septum in the port area (3.2).

5. A cap according to any of the preceding claims, **characterized in that** the flexible port area (3.2) is designed in the style of a sheet.

6. A cap according to any of the preceding claims, **characterized in that** the port area (3.2) has a radius of curvature r_d relative to a diameter d_p of the flexible port area according to the formula: r_d > d_p/2.

7. A cap according to any of the preceding claims, **characterized in that** the septum (3) is a body of silicone (polysiloxane), preferably with a circular shape.

8. A cap according to claim 5, **characterized in that** the sheet body has a bulge (3.7) at a peripheral rim (3.6) of the sheet body, the bulge (3.7) protruding substantially orthogonal to a plane (3.9) defined by the periphery of the sheet body of the septum and improving the fixation in the receiving channel (2.6).

9. A cap according to any of claims 5 and 6, **characterized in that** the septum has a transition section (3.3) between the rim (3.6) and the port area (3.2).

10. A cap according to any of the preceding claims, **characterized in that** the dome has a height that is not more than 1/5 of a diameter of the dome.

11. A cap according to any of the preceding claims, **characterized in that** the port area (3.2) is defined by slits (3.1) in the septum, the slits (3.1) dividing the port area into flaps (3.11 - 3.14) that give way to the conduit (4).

12. A cap according to any of the preceding claims, **characterized in that** the cap (3) has a peripheral wall (2.1) oriented orthogonal to the closure face (2.2), the peripheral wall (2.1) running along a peripheral edge of the closure face (2.2) and having structural elements (2.5) to mechanically fix the cap (2) at the container opening (1.3).

13. A cap according to any of the preceding claims, **characterized in that** the closure face (2.2) has a rigid structure, the rigid structure having an opening (2.3) limited by a surrounding edge in which the septum (3) is fixed, wherein the peripheral rim (3.6) of the septum (3) is fixed in a u-shaped receiving channel (2.6) provided in the surrounding edge of the opening (2.3).

14. A cap according to the preceding claim, **characterized in that** the u-shaped receiving channel is open towards a center of the opening.

15. A cap according to any of the preceding claims, **characterized in that** the rigid structure of the cap is made of a stiff plastic material, preferably of a polyethylene.
